# EUROPÄISCHE PATENTSCHRIFT

(11) **EP 3 421 895 B1**
(45) Veröffentlichungstag und Bekanntmachung des Hinweises auf die Patenterteilung: **18.11.2020**
(21) Anmeldenummer: 17177848.3
(22) Anmeldetag: 26.06.2017
(51) Int. Cl.: F24F 3/12, B60H 3/00, F24F 3/16

(54) **LUFTAUFBEREITUNGSEINRICHTUNG ZUM EINSATZ IN EINEM LUFTSTRÖMUNGSPFAD EINES KLIMATISIERUNGSSYSTEMS IN EINER MOBILEN EINRICHTUNG**
AIR PREPARATION DEVICE FOR USE IN AN AIR FLOW PATH OF AN AIR-CONDITIONING SYSTEM IN A MOBILE DEVICE
DISPOSITIF DE PRÉPARATION D'AIR DESTINÉ À ÊTRE UTILISÉ DANS UN CHEMIN D'ÉCOULEMENT D'AIR D'UN SYSTÈME DE CLIMATISATION DANS UN DISPOSITIF MOBILE

(43) Veröffentlichungstag der Anmeldung: 02.01.2019
(73) Patentinhaber: Carl Freudenberg KG, 69469 Weinheim (DE)
(72) Erfinder: Stahl, Ulrich, 69514 Laudenbach (DE); Bräunling, Volker, 64646 Heppenheim (DE)

(56) Entgegenhaltungen:
- EP-A1- 0 645 148
- EP-A1- 0 720 925
- DE-A1-102007 044 978
- DE-A1-102009 058 111
- DE-A1-102013 015 649
- US-A1- 2006 005 711

## Beschreibung

### Technisches Gebiet

Die Erfindung betrifft Luftaufbereitungseinrichtungen zur Anordnung in einer Luftströmung zur Klimatisierung eines Innenraums, insbesondere eines Innenraums einer mobilen Einrichtung, wie z.B. einem Kraftfahrzeug.

### Technischer Hintergrund

Luftaufbereitungseinrichtungen können dazu dienen, Klimatisierungsluft mit einem Duft zu versehen, bevor diese in einen Innenraum geführt wird. Oft ist eine derartige Luftaufbereitungseinrichtung mit einem mit Duftstoff getränkten Vliesstoff ausgebildet, der im Strömungspfad angeordnet ist. Ein Lüfter fördert dabei den Luftstrom durch den Vliesstoff in Richtung des Innenraumes.

Die Kapazität eines mit Duftstoffen getränkten Vliesstoffes ist sehr eingeschränkt, so dass die Duftwirkung nur kurz andauern kann. Auch ist eine Regelung der Beduftungsintensität mit diesem System nicht möglich. So nimmt die Beduftungswirkung über die Zeit erheblich ab.

Außerdem stellen derartige Luftaufbereitungseinrichtungen einen Strömungswiderstand dar, durch den die Strömungsstärke des Luftstroms reduziert wird und wodurch für einen vorgegebenen Klimatisierungsluftstrom eine erhöhte elektrische Lüfterleistung benötigt wird. Diese führt jedoch zu einer zusätzlichen Geräuschentwicklung, das störend im Innenraum wahrgenommen werden kann.

Weiterhin sind mit granularen Duftstoffen gefüllte Duftkartuschen bekannt, die im Strömungsbereich in den Luftführungskanälen oder in den Faltungen eines Luftfilters angeordnet sind. Diese Duftkartuschen stören die Luftführung, erzeugen ungewünschte Luftturbulenzen und können ebenfalls erhöhte Strömungsgeräusche verursachen.

Die Druckschrift EP 0720925A1 offenbart einen Luftauffrischer mit einem Duftgeber und einem Befestigungsmittel, wobei eine im wesentlichen ebene Basisplatte und eine parallel und beabstandet von der Basisplatte angeordnete, im wesentlichen ebene Deckplatte, wobei der Duftgeber zwischen der Basisplatte und der Deckplatte angeordnet ist und wobei das Befestigungsmittel an der Basisplatte angeordnet ist. Druckschrift EP 0645148 A1 offenbart eine Klimatisierungsvorrichtung, umfassend ein Gehäuse mit einem Luftströmungsweg, Gebläseeinrichtungen zum Bewegen von Luft entlang des Strömungswegs, Mittel zum Verteilen einer Klimatisierungssubstanz in die sich bewegende Luft.

Aus der Druckschrift DE 10 2009 058 111 A1 ist ein Luftauslass für eine Belüftungseinrichtung eines Kraftfahrzeugs bekannt, mit wenigstens einer Austrittsöffnung, über welche ein Fahrgastraum des Kraftfahrzeugs mit Luft beaufschlagbar ist, wobei der Luftauslass einen Aufnahmeraum aufweist, in welchen ein Duftstoffträger zum Beaufschlagen der Luft mit einem Duftstoff zumindest bereichsweise einbringbar ist.

Allgemein wird das Vorsehen einer Beduftung der Klimatisierungsluft mit einer zusätzlichen Luftaufbereitungseinrichtung erreicht, das einen zusätzlichen Platzbedarf im Klimatisierungssystem erfordert und einen zusätzlichen Aufwand bei der Montage darstellt.

Es ist daher Aufgabe der vorliegenden Erfindung, eine verbesserte Möglichkeit bereitzustellen, in einem Klimatisierungssystem die in einen Innenraum zugeführte Klimatisierungsluft mit Duftstoffen anzureichern.

### Offenbarung der Erfindung

Diese Aufgabe wird durch die Luftaufbereitungseinrichtung für ein Klimatisierungssystem gemäß Anspruch 1 sowie das Klimatisierungssystem gemäß dem nebengeordneten Anspruch gelöst.

Weitere Ausgestaltungen sind in den abhängigen Ansprüchen angegeben.

Gemäß einem ersten Aspekt ist eine Luftaufbereitungseinrichtung für eine Luftströmung eines Klimatisierungssystems vorgesehen, umfassend ein Luftaufbereitungselement mit einer oder mehreren sich in einer Strömungsrichtung einer Klimatisierungsluft erstreckenden Durchgangsöffnungen, wobei die Durchgangsöffnungen in Strömungsrichtung verlaufende Beduftungsflächen aufweisen, die ausgebildet sind, einen oder mehrere Duftstoffe an eine durchströmende Luftströmung abzugeben.

Weiterhin weist das Aufbereitungselement einen Mehrkomponenten-Vliesstoff, insbesondere einen Spinnvliesstoff oder einen Stapelfaservliesstoff, auf wobei der Mehrkomponenten-Vliesstoff Fasern aufweist, die einen Kernbereich und einen den Kernbereich umgebenden Außenbereich aufweisen. Der Kernbereich und der Außenbereich unterscheiden sich in ihren chemischen und/oder physikalischen Eigenschaften. Mindestens einer der Duftstoffe ist ausschließlich in dem Außenbereich der Fasern des Vliesstoffes eingebettet.

Eine Idee der obigen Luftaufbereitungseinrichtung besteht darin, die Funktion der Strömungsgleichrichtung, des Beduftens, einer Ozon-Reduktion und einer geräuschmindernden Wirkung gleichzeitig zu erreichen. Dabei stellt die Luftaufbereitungseinrichtung ein in ein Klimatisierungssystem einzusetzendes Bauteil mit im Wesentlichen einem einzelnen Element dar, durch das die Klimatisierungsluft aufbereitet werden kann. Die Luftaufbereitungseinrichtung weist dazu im Wesentlichen ein Luftaufbereitungselement mit entlang der Strömungsrichtung verlaufenden Beduftungsflächen auf, die mit einem Duftstoff angereichert sind, so dass bei Durchströmen der Klimatisierungsluft diese mit dem Duftstoff angereichert wird.

Durch das Vorsehen eines möglichst großen Flächenanteils, an dem die Luftströmung entlangströmt, können stromaufwärts erzeugte Geräusche, wie beispielsweise ein Lüftergeräusch, durch eine große Anzahl von Schallreflexionsflächen gebrochen werden, so dass eine erhebliche Geräuschreduzierung erreicht werden kann, ohne dass zusätzliche Geräusche durch das Entlangströmen der Klimatisierungsluft entlang der Beduftungsflächen erzeugt werden. Weiterhin kann die Luftströmung durch eine parallele Anordnung der Beduftungsflächen entlang deren Verlaufs gleichgerichtet werden, um so beim Eintritt der Klimatisierungsluft in den Innenraum einen laminareren Strömungsverlauf zu erreichen. Auch besteht bei einer solchen Anordnung der Beduftungsflächen ein geringerer Strömungswiderstand, der wiederum zu einer geringeren Geräuschentwicklung in dem Klimatisierungssystem führt. Zudem wird erreicht, dass aufgrund der großen Fläche, an der die Klimatisierungsluft entlangströmt, das in der Klimatisierungsluft befindliche Ozon reduziert wird.

Somit kann durch die Luftaufbereitungseinrichtung eine Strömungsgleichrichtung, eine Beduftungsfunktion, eine Ozonreduktion und eine geräuschmindernde Wirkung mit einem gemeinsamen Bauelement erreicht werden.

Gemäß einer Ausführungsform kann das Aufbereitungselement einen Vliesstoff, insbesondere einen Spinnvliesstoff oder einen Stapelfaservliesstoff, aufweisen und mindestens einer der Duftstoffe in Fasern des Vliesstoffes eingebettet sein.

Weiterhin können die Durchgangsöffnungen des Aufbereitungselements einen wabenförmigen, kreisförmigen, ovalen, rechteckigen und mehreckigen Querschnitt aufweisen.

Es kann vorgesehen sein, dass das Aufbereitungselement einen Querschnitt aufweist, der durch flächige Lamellen ausgebildet ist und insbesondere einen sternförmigen Querschnitt aufweist.

Gemäß einer Ausführungsform kann das Aufbereitungselement aus einem Kunststoff ausgebildet sein, wobei mindestens einer der Duftstoffe als verkapselter Duftstoff in eine Polymermatrix des betreffenden Kunststoffs des Elementkörpers des Aufbereitungselements eingebracht ist.

Gemäß einer weiteren Ausführungsform kann das Luftaufbereitungselement mit einem Mehrkomponenten-Kunststoff ausgebildet sein, wobei mindestens einer der Duftstoffe als verkapselter Duftstoff in eine Polymermatrix des ersten Kunststoffes des Elementkörpers des Luftaufbereitungselements eingebracht ist, wobei der erste Kunststoff gezielt an der Oberfläche der Beduftungsflächen des Luftaufbereitungselements angeordnet ist und mindestens ein zweiter Kunststoff (d.h. z.B. ein anderes Kunststoffmaterial, eine andere Materialstruktur oder dergleichen) im Kern des Aufbereitungselements angebracht ist. Durch die oberflächennahe Anordnung des Duftstoffes wird die Abgabe an den Luftstrom verbessert und die Menge des verwendeten Duftstoffes kann reduziert werden.

Es kann vorgesehen sein, dass das Luftaufbereitungselement mit einer Heizeinrichtung versehen ist, um durch Erwärmen des Aufbereitungselements die Rate der Abgabe des einen oder der mehreren Duftstoffe zu steuern.

Insbesondere kann die Konzentration des in den Luftstrom abgegebenen Duftstoffes durch die Partialdruckdifferenz/Konzentration der Duftstoffe innerhalb des Elementes gegenüber der umgebenden Atmosphäre und dem Temperaturgradient zwischen Element und Umgebung erreicht werden.

### Kurzbeschreibung der Zeichnungen

Ausführungsformen werden nachfolgend anhand der beigefügten Zeichnungen näher erläutert. Es zeigen:
- Figur 1: eine schematische Darstellung eines Klimatisierungssystems für einen Innenraum; und
- Figuren 2a und 2b: eine detailliertere Darstellung einer möglichen Ausführungsform einer Aufbereitungseinrichtung in einer Seitenansicht und in einer Querschnittsansicht;
- Figur 3: eine detaillierte Darstellung einer Querschnittsansicht einer weiteren Aufbereitungseinrichtung;
- Figur 4: eine Querschnittsansicht eines weiteren Aufbereitungselements; und
- Figuren 5a und 5b: eine Querschnittsansicht eines Vliesstoffes als Aufbereitungselement und einer vergrößerten Abbildung eines Schnittes durch den Vliesstoff.

### Beschreibung von Ausführungsformen

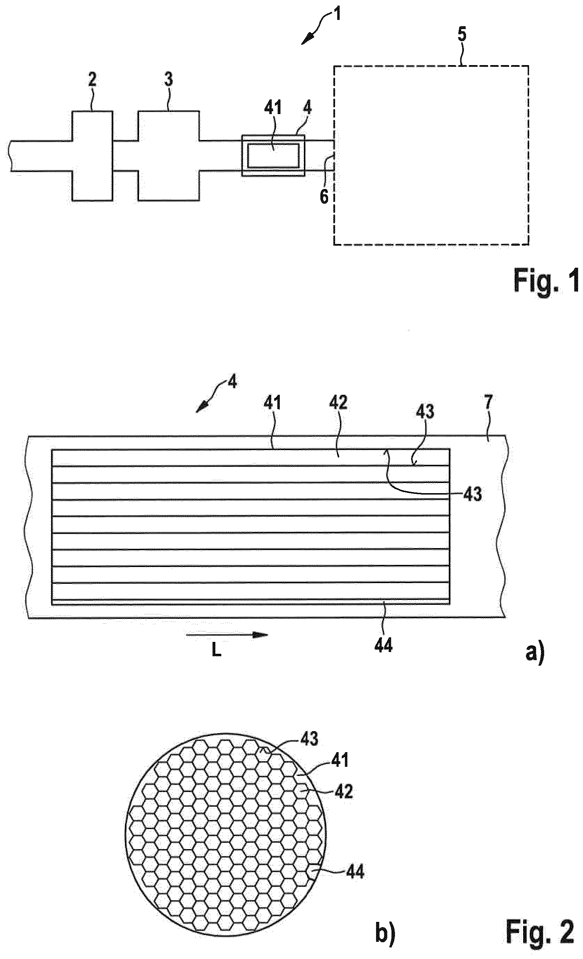

Figur 1 zeigt schematisch ein Klimatisierungssystem 1 für einen Innenraum, z. B. einen Innenraum eines Kraftfahrzeugs oder dergleichen. Das Klimatisierungssystem 1 umfasst einen Lüfter 2, um Frischluft aus der Umgebung anzusaugen und eine Luftströmung bereitzustellen, die anschließend durch einen Wärmetauscher 3 zum Kühlen oder Erwärmen der Klimatisierungsluft geführt wird.

Stromabwärts des Wärmetauschers 3 ist eine Luftaufbereitungseinrichtung 4 in der Luftströmung vor dem Eintritt der Luftströmung in den Innenraum 5 angeordnet. Insbesondere ist die Luftaufbereitungseinrichtung 4 unmittelbar an einem Luftauslass 6 oder möglichst nah an dem Luftauslass 6 in dem Innenraum 5 angeordnet und kann austauschbar in der Luftströmung angeordnet sein.

Die Figuren 2a und 2b zeigen verschiedene Ansichten einer beispielhaften Luftaufbereitungseinrichtung 4. In Figur 2a ist eine Schnittansicht entlang der Längsrichtung L gezeigt, die der Strömungsrichtung der Klimatisierungsluft entspricht. Figur 2b zeigt eine entsprechende Querschnittsansicht. Figur 2a zeigt die Luftaufbereitungseinrichtung 4 mit einem Aufbereitungselement 41, das in einem Strömungspfad 7 des Klimatisierungssystems 1 angeordnet ist. Das Aufbereitungselement 41 weist eine Vielzahl von sich entlang des Strömungspfades 7 erstreckenden Durchgangsöffnungen 42 auf, deren Innenflächen im Wesentlichen parallel zueinander verlaufen. Die Innenflächen der Durchgangsöffnungen 42 können ganz oder teilweise als Beduftungsflächen 43 ausgebildet sein.

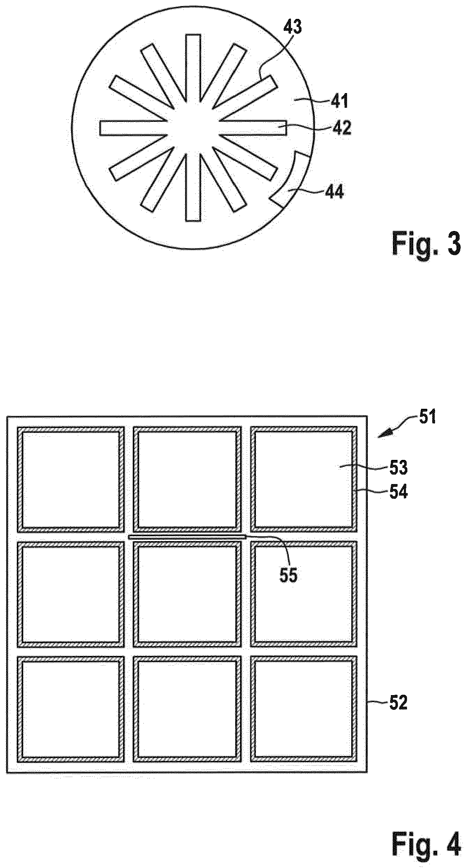

Der Elementkörper des Aufbereitungselements 41 kann anstelle der Kanalstruktur auch im Strömungspfad angeordnete Lamellen umfassen, wie beispielsweise in der Querschnittsansicht der Figur 3 dargestellt, sternförmig angeordnete Lamellen 42, deren Außenflächen sich parallel zum Strömungspfad erstrecken und ganz oder teilweise als Beduftungsflächen 43 ausgebildet sind.

Im Bereich der Beduftungsflächen 43 können die Oberflächen mit einem Duftstoff beaufschlagt sein oder verkapselte Duftstoffe beinhalten.

Die Durchgangsöffnungen 42 des Aufbereitungselements 41 können im Querschnitt eine Wabenstruktur, einen kreisförmigen Querschnitt oder eine sonstige Querschnittsflächenform aufweisen. Das Aufbereitungselement 41 kann eine Länge in Strömungsrichtung von zwischen 1 bis 10 cm aufweisen. Insbesondere kann das Verhältnis aus Querschnittsfläche Q [cm²] und der Länge L[cm] des Aufbereitungselements 41 L/Q = 0,1 oder weniger betragen.

Die Figur 4 zeigt als ein Beispiel eine Querschnittsansicht eines weiteren Aufbereitungselements 51, das als ein Mehrkomponenten-Spritzguss Kunststoffelement ausgebildet ist. Es weist einen Elementkern 52 auf, der aus einem ersten Kunststoffmaterial ausgebildet ist, der nicht oder nur teilweise mit Duftstoffen versehen ist. Der Elementkern 52 weist Durchgangsöffnungen 53 auf, die im vorliegenden Ausführungsbeispiel einen quadratischen Querschnitt aufweisen, aber nicht auf diese Form des Querschnittes beschränkt sind. So können auch andere Querschnittsformen vorgesehen und zweckmäßig sein.

Die Oberflächen in den Durchgangsöffnungen 53 sind mit einer Beschichtung 54 oder Lage mit einem zweiten Kunststoffmaterial versehen. Das zweite Kunststoffmaterial ist in einer der oben beschriebenen Arten mit Duftstoffen versehen, wobei mindestens einer der Duftstoffe nur in dem zweiten Kunststoffmaterial vorgesehen ist. Dadurch kann der mindestens eine Duftstoff nahe an der Oberfläche platziert werden, wodurch die Effizienz des Einsatzes des mindestens einen Duftstoffes verbessert werden kann.

Der Elementkörper des Aufbereitungselements 41 kann aus einem Material hergestellt sein, das mehrheitlich aus einem Kunststoff, wie zum Beispiel PP, PES, PA und dergleichen ausgebildet ist. Das Luftaufbereitungselement 41 ist insbesondere an dessen Oberflächen mit einem oder mehreren Duftstoffen versehen, der an die durchströmende Luftströmung abgegeben werden kann. Dazu kann der bzw. die Duftstoffe als verkapselte Duftstoffe in die Polymermatrix des betreffenden Kunststoffs des Elementkörpers eingebracht werden. Die Duftstoffe werden daraus über die Zeit an die Luftströmung abgegeben und mit dieser in den Innenraum 5 getragen.

Als Duftstoffe kommen handelsübliche "aromatische" Substanzen zum Einsatz, die während des Herstellungsprozesses des Elementkörpers in einem Formgebungsprozess (z.B. Spritzguss, Extrusion oder Faserspinnen) verkapselt in die Materialstruktur des Elementes eingebracht wird. Als Duftstoffe können z.B. Cumarin, Benzylidenheptanal, Limone, Eugenol, 2-Phenyläthylalkohol, Heliotropin, Vanillin, Muscon und Maltol verwendet werden.

Die gleichmäßige Verteilung der in einer Polymermatrix verkapselten Duftstoffe im Elementkörper wird durch den Herstellungsprozess, hier insbesondere den Spritzgussprozess oder Extrusionsprozess im Falle eines Wabenkörpers oder den Faserspinnprozess im Falle eines Vliesstoffelementes bestimmt.

Der Austrag des bzw. der Duftstoffe von den Oberflächen der Durchgangsöffnungen 42 oder aus dem Material des Aufbereitungselements 41 kann durch eine steuerbare Heizeinrichtung 44 gesteuert werden, wobei die Konzentration der Duftstoffe bei höheren Temperaturen erhöht wird. Die Heizeinrichtung 44 kann vorzugsweise als elektrische Heizeinrichtung ausgebildet sein, die insbesondere temperaturgeregelt sein kann. Die Heizeinrichtung 44 kann am Rand des Elementkörpers oder mittig des Elementkörpers vorzugsweise sich über die Länge des Elementkörpers erstreckend vorgesehen sein.

Durch die Ausbildung des Aufbereitungselements 41 mit einer Vielzahl von einzelnen Durchgangsöffnungen 42 kann die Fläche, an der die Luftströmung der Klimatisierungsluft entlangstreicht, erheblich vergrößert werden, so dass die Aufnahme von Duftstoffen aus dem Elementkörper erleichtert wird.

Weiterhin führt eine große Oberfläche, an der die Luftströmung entlangströmt, zu einer Reduzierung von in der durchströmenden Klimatisierungsluft enthaltenen Ozon, das in der Regel durch Kontakt an beliebigen Oberflächen reduziert wird.

Auch erfolgt eine Strömungsgleichrichtung, indem Turbulenzen in dem Luftstrom reduziert werden und die ausgangsseitig der Luftaufbereitungseinrichtung 4 austretende Luftströmung laminarisiert ist. Dies reduziert die Strömungsgeräusche ausgangsseitig der Aufbereitungseinrichtung 4.

Figur 4 zeigt als ein Beispiel eine Querschnittsansicht eines weiteren Aufbereitungselements 51, das als ein Mehrkomponenten-Spritzguss Kunststoffelement oder Mehrkomponenten-Extrusionselement ausgebildet ist. Es weist einen Elementkern 52 auf, der aus einem ersten Kunststoffmaterial ausgebildet ist, der nicht mit Duftstoffen versehen ist. Der Elementkern 52 weist Durchgangsöffnungen 53 auf. Die Durchgangsöffnungen 53 sind im vorliegenden Ausführungsbeispiel mit einem rechteckigen Querschnitt versehen. Auch andere Querschnitte der Durchgangsöffnungen 53 für das Mehrkomponenten-Kunststoffelement können vorgesehen werden.

Die Oberflächen in den Durchgangsöffnungen 53 sind mit einer Beschichtung 54 oder Lage mit einem zweiten Kunststoffmaterial versehen. Das zweite Kunststoffmaterial ist in einer der oben beschriebenen Arten mit Duftstoffen versehen. Dadurch können die Duftstoffe nahe an der Oberfläche platziert werden können, wodurch die Effizienz des Einsatzes von Duftstoffen verbessert werden kann.

In dem Elementkern 52 kann eine Heizeinrichtung 55, wie oben beschrieben, eingebettet sein.

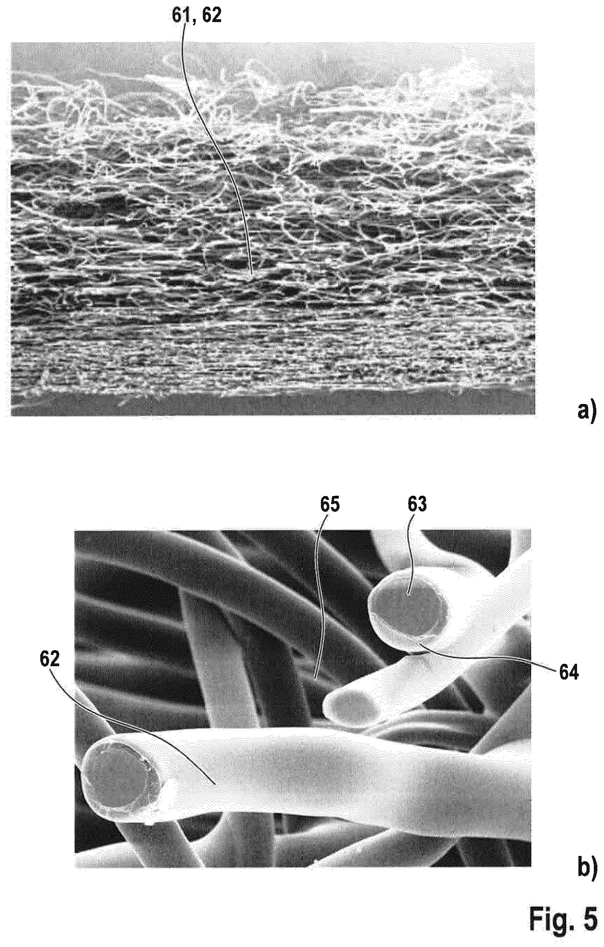

Figur 5 zeigt als ein Beispiel eine Querschnittsansicht eines weiteren Aufbereitungselements 61, das als ein Mehrkomponenten-Vliesstoffelement ausgebildet ist. Das Mehrkomponenten-Vliesstoffelement 61 besteht aus Fasern 62, welche einen Elementkern 63 aufweisen, der aus einem ersten Kunststoffmaterial ausgebildet ist, der nicht oder nur teilweise mit Duftstoffen versehen ist. Das zweite Kunststoffmaterial 64 ist in der Querschnittsansicht der Faser als äußerer Mantel dargestellt, der die Fasern 62 vollständig umgibt. Alternativ kann das zweite Kunststoffmaterial die Fasern 62 nur teilweise, d.h. nicht vollumfänglich bedecken. Die Beduftungsflächen in den Durchgangsöffnungen 65 entsprechen den durch das zweite Kunststoffmaterial 64 bedeckten Flächen bzw. der Flächen des Elementkerns 63, die mit dem zweiten Kunststoffmaterial beschichtet sind. Das zweite Kunststoffmaterial 64 ist in einer der oben beschriebenen Arten mit Duftstoffen versehen. Dadurch können die Duftstoffe nahe an der Oberfläche platziert werden, wodurch die Effizienz des Einsatzes von Duftstoffen verbessert werden kann.

Die Durchgangsöffnungen 65 des Aufbereitungselements 61 können im Querschnitt inhomogen und variabel angeordnet sein und entsprechen den freien Zwischenräumen zwischen den Fasern. Das Aufbereitungselement 61 kann eine Länge in Strömungsrichtung von zwischen 1 bis 5 cm aufweisen. Insbesondere kann das Verhältnis aus Querschnittsfläche Q [cm²] und der Länge L[cm] des Aufbereitungselements 61 L/Q = 0,05 oder weniger betragen.

Das Mehrkomponenten-Vliesstoffelement kann aus einem oder mehreren Kunststoffen der Materialklassen PP, PES, PET, PA, PBT bestehen, wobei das erste und das zweite Kunststoffmaterial sich auch nur in ihren physikalischen Eigenschaften, wie z.B. Temperaturbeständigkeit, Schmelzpunkt oder Erweichungspunkt unterscheiden können.

### Bezugszeichenliste

- 1: Klimatisierungssystem
- 2: Lüfter
- 3: Wärmetauscher
- 4: Luftaufbereitungseinrichtung
- 5: Innenraum
- 6: Luftauslass
- 41: Aufbereitungselement
- 42: Durchgangsöffnungen
- 43: Beduftungsflächen
- 44: steuerbare Heizeinrichtung
- 51: Aufbereitungselement
- 52: Elementkern
- 53: Durchgangsöffnungen
- 54: Beschichtung
- 55: Heizeinrichtung
- 61: Aufbereitungselement
- 62: Fasern
- 63: Faserkern
- 64: Faseraußenbereich
- 65: Durchgangsöffnungen
- 7: Strömungspfad

## Patentansprüche

1. Luftaufbereitungseinrichtung (4) für eine Luftströmung eines Klimatisierungssystems (1), umfassend ein Aufbereitungselement (41, 51) mit einer oder mehreren sich teilweise oder vollständig in einer Strömungsrichtung erstreckenden Durchgangsöffnungen (42, 53, 65), wobei die Durchgangsöffnungen (42, 53, 65) in Strömungsrichtung verlaufende Beduftungsflächen (43) aufweisen, die ausgebildet sind, einen oder mehrere Duftstoffe an eine durchströmende Luftströmung abzugeben, **dadurch gekennzeichnet, dass** das Aufbereitungselement (41, 51) einen Mehrkomponenten-Vliesstoff, insbesondere einen Spinnvliesstoff oder einen Stapelfaservliesstoff, aufweist, wobei der Mehrkomponenten-Vliesstoff Fasern aufweist, welche einen Kernbereich und einen den Kernbereich umgebenden Außenbereich aufweisen, welche sich in ihren chemischen und/oder physikalischen Eigenschaften unterscheiden und wobei mindestens einer der Duftstoffe ausschließlich in den Außenbereich der Fasern des Vliesstoffes eingebettet ist.

2. Luftaufbereitungseinrichtung (4) nach Anspruch 1, wobei das Aufbereitungselement (41, 51) einen Vliesstoff, insbesondere einen Spinnvliesstoff oder einen Stapelfaservliesstoff, aufweist und mindestens einer der Duftstoffe in den Fasern des Vliesstoffes eingebettet ist.

3. Luftaufbereitungseinrichtung (4) nach Anspruch 1, wobei die Durchgangsöffnungen (42, 53) des Aufbereitungselements (41) jeweils einen wabenförmigen, kreisförmigen, ovalen, und mehreckigen Querschnitt aufweisen.

4. Luftaufbereitungseinrichtung (4) nach Anspruch 1, wobei das Aufbereitungselement (41) einen Querschnitt aufweist, der durch flächige Lamellen ausgebildet ist und insbesondere einen sternförmigen Querschnitt aufweist.

5. Luftaufbereitungseinrichtung (4) nach einem der Ansprüche 3 bis 4, wobei das Aufbereitungselement (41, 51) aus einem Kunststoff ausgebildet ist, mindestens einer der Duftstoffe als verkapselter Duftstoff in eine Polymermatrix des betreffenden Kunststoffs des Elementkörpers des Aufbereitungselements (41, 51) eingebracht ist.

6. Luftaufbereitungseinrichtung (4) nach einem der Ansprüche 3 bis 4, wobei das Aufbereitungselement (51, 61) mit einem Mehrkomponenten-Kunststoff ausgebildet ist und mindestens einer der Duftstoffe als verkapselter Duftstoff in eine Polymermatrix eines ersten Kunststoffs des Elementkörpers des Aufbereitungselements (51, 61) eingebracht ist, wobei der erste Kunststoff gezielt an der Oberfläche der Beduftungsflächen (43) des Aufbereitungselements (51, 61) angeordnet ist und mindestens ein zweiter Kunststoff im Kern des Aufbereitungselements (41, 51) angebracht ist.

7. Luftaufbereitungseinrichtung (4) nach einem der Ansprüche 1 bis 6, wobei das Aufbereitungselement (41, 51, 61) mit einer Heizeinrichtung (44, 55) versehen ist, um durch Erwärmen des Aufbereitungselements (41, 51, 61) die Rate der Abgabe des einen oder der mehreren Duftstoffe zu steuern.

8. Klimatisierungssystem (1) für einen Innenraum, insbesondere einen Innenraum eines Kraftfahrzeuges, mit einer Luftaufbereitungseinrichtung (4) nach einem der Ansprüche 1 bis 7, die in einer Luftströmung des Klimatisierungssystems (1) angeordnet ist.

9. Klimatisierungssystem (1) nach Anspruch 8, wobei das Klimatisierungssystem (1) einen Luftauslass (6) aufweist, wobei die Luftaufbereitungseinrichtung (4) stromaufwärts des Luftauslasses (6) angeordnet ist und insbesondere austauschbar gehalten ist.

## Claims

1. Air treatment device (4) for an airflow of an air conditioning system (1), comprising a treatment element (41, 51) having one or more passages (42, 53, 65) extending in part or completely in a flow direction, the passages (42, 53, 65) comprising fragrancing areas (43) that extend in the flow direction and are designed to release one or more fragrant substances into an airflow that is flowing through, **characterized in that** the treatment element (41, 51) comprises a multicomponent nonwoven, in particular a spunbonded nonwoven or a staple fibre nonwoven, the multicomponent nonwoven comprising fibres which have a core region and an outer region surrounding the core region, which differ in their chemical and/or physical properties, and at least one of the fragrant substances being embedded in the outer region of the fibres of the nonwoven.

2. Air treatment device (4) according to Claim 1, the treatment element (41, 51) comprising a nonwoven, in particular a spunbonded nonwoven or a staple fibre nonwoven, and at least one of the fragrant substances being embedded in the fibres of the nonwoven.

3. Air treatment device (4) according to Claim 1, the passages (42, 53) of the treatment element (41) each having a honeycomb-shaped, circular, oval and polygonal cross section.

4. Air treatment device (4) according to Claim 1, the treatment element (41) having a cross section formed by planar slats, and in particular having a star-shaped cross section.

5. Air treatment device (4) according to one of Claims 3 to 4, the treatment element (41, 51) being formed from a plastic, and at least one of the fragrant substances being incorporated as an encapsulated fragrant substance in a polymer matrix of the plastic concerned of the element body of the treatment element (41, 51).

6. Air treatment device (4) according to one of Claims 3 to 4, the treatment element (51, 61) being formed from a multicomponent plastic and at least one of the fragrant substances being incorporated as an encapsulated fragrant substance in a polymer matrix of a first plastic of the element body of the treatment element (51, 61), the first plastic being arranged specifically on the surface of the fragrancing areas (43) of the treatment element (51, 61) and at least a second plastic being provided in the core of the treatment element (41, 51).

7. Air treatment device (4) according to one of Claims 1 to 6, the treatment element (41, 51, 61) being provided with a heating device (44, 55) in order to control the rate of release of the one or more fragrant substances by heating the treatment element (41, 51, 61).

8. Air conditioning system (1) for an interior space, in particular a passenger compartment of a motor vehicle, comprising an air treatment device (4) according to one of Claims 1 to 7, which is arranged in an airflow of the air conditioning system (1).

9. Air conditioning system (1) according to Claim 8, the air conditioning system (1) comprising an air outlet (6), the air treatment device (4) being arranged upstream of the air outlet (6) and in particular mounted so as to be exchangeable.

## Revendications

1. Dispositif de traitement d'air (4) destiné à un flux d'air d'un système de climatisation (1), ledit dispositif comprenant un élément de traitement (41, 51) pourvu d'une ou de plusieurs ouvertures de passage (42, 53, 65) s'étendant partiellement ou complètement dans une direction d'écoulement, les ouvertures de passages (42, 53, 65) comportant des surfaces parfumantes (43) qui s'étendent dans la direction d'écoulement et qui sont conçues pour délivrer un ou plusieurs parfums à un flux d'air, **caractérisé en ce que** l'élément de traitement (41, 51) comporte un non-tissé à plusieurs composants, en particulier un non-tissé filé-lié ou un non-tissé à fibres discontinues, le non-tissé à plusieurs composants comportant des fibres qui comportent une zone centrale et une zone extérieure entourant la zone centrale, lesquelles zones différant par leurs propriétés chimiques et/ou physiques et au moins un des parfums étant incorporé exclusivement dans la zone extérieure des fibres du non-tissé.

2. Dispositif de traitement d'air (4) selon la revendication 1, l'élément de traitement (41, 51) comportant un non-tissé, en particulier un non-tissé filé-lié ou un non-tissé à fibres discontinues, et l'un au moins des parfums étant incorporé dans les fibres du non-tissé.

3. Dispositif de traitement d'air (4) selon la revendication 1,
les ouvertures de passage (42, 53) de l'élément de traitement (41) ayant chacune une section transversale en nid d'abeille, circulaire, ovale et polygonale.

4. Dispositif de traitement d'air (4) selon la revendication 1, l'élément de traitement (41) ayant une section transversale qui est formée par des lamelles sensiblement bidimensionnelles et qui a en particulier une section transversale en forme d'étoile.

5. Dispositif de traitement d'air (4) selon l'une des revendications 3 à 4, l'élément de traitement (41, 51) étant formé à partir d'une matière synthétique, l'un au moins des parfums étant introduit sous forme de parfum encapsulé dans une matrice polymère de la matière synthétique concernée du corps de l'élément de traitement (41, 51).

6. Dispositif de traitement d'air (4) selon l'une des revendications 3 à 4, l'élément de traitement (51, 61) étant conçu avec une matière synthétique à plusieurs composants et l'un au moins des parfums étant introduit sous forme de parfum encapsulé dans une matrice polymère d'une première matière synthétique du corps de l'élément de traitement (51, 61), la première matière synthétique étant disposée spécifiquement sur la surface des surfaces parfumantes (43) de l'élément de traitement (51, 61) et au moins une deuxième matière synthétique étant fixée dans le noyau de l'élément de traitement (41, 51).

7. Dispositif de traitement d'air (4) selon l'une des revendications 1 à 6, l'élément de traitement (41, 51, 61) étant pourvu d'un dispositif de chauffage (44, 55) pour commander la vitesse de libération du ou des parfums par chauffage de l'élément de traitement (41, 51, 61) .

8. Système de climatisation (1) destiné à un espace intérieur, notamment un espace intérieur de véhicule automobile et comprenant un dispositif de traitement d'air (4) selon l'une des revendications 1 à 7 qui est disposé dans un flux d'air du système de climatisation (1) .

9. Système de climatisation (1) selon la revendication 8, le système de climatisation (1) comportant une sortie d'air (6), le dispositif de traitement d'air (4) étant disposé en amont de la sortie d'air (6) et étant en particulier maintenu de manière remplaçable.
